# EUROPEAN PATENT APPLICATION

(11) **EP 3 269 292 A1**
(43) Date of publication of application: **17.01.2018**
(21) Application number: 16866312.8
(22) Date of filing: 15.11.2016
(51) Int. Cl.: A61B 1/00

(54) **ENDOSCOPE**

(30) Priority: 17.11.2015 JP 2015224987
(71) Applicant: Olympus Corporation, Hachioji-shi, Tokyo 192-8507 (JP)
(72) Inventor: OKANIWA Suguru, Tokyo 192-8507 (JP); TAKASE Seisuke, Tokyo 192-8507 (JP); JOKO Hidehiro, Tokyo 192-8507 (JP); NAKAJIMA Isamu, Tokyo 192-8507 (JP)
(74) Representative: Schicker, Silvia
(86) International application number: PCT/JP2016/083817
(87) International publication number: WO 2017/086311

(57) **Abstract**

An endoscope includes: a flexible tube portion provided to an insertion portion that extends from a hand side to a distal end side along a longitudinal direction; a coil pipe arranged in the flexible tube portion, and formed by being wound around a predetermined axis that is parallel to the longitudinal direction; a first wire inserted in the coil pipe and twined around the predetermined axis in a direction opposite to a winding direction of the coil pipe, a distal end side of the first wire being fixed to a distal end of the coil pipe; a second wire formed by being twined in a direction opposite to a twining direction of the first wire, a proximal end of the second wire being fixed to a portion at which the first wire and the coil pipe are fixed to each other, and a distal end of the second wire being engaged with a constituent element of the endoscope with rotation of the distal end being restricted; and a pulling mechanism portion that pulls the first wire in a proximal end direction to apply a compression force to the coil pipe.

## Description

### Technical Field

The present invention relates to an endoscope including a rigidity changing mechanism portion in an insertion portion.

### Background Art

Endoscopes have been used in medical fields and industrial fields, for example, and such endoscopes include an image pickup unit for picking up an optical image in a distal end portion of an insertion portion configured to be insertable from an outside to an inside of a living body or structure, in order to observe inside the living body or the structure where observation is difficult to perform.

The endoscope disclosed in Japanese Patent Application Laid-Open Publication No. 10-276965 has a rigidity changing mechanism portion that changes rigidity in a bending direction of a part of an insertion portion. The rigidity changing mechanism portion includes a coil pipe inserted in the insertion portion, a wire inserted in the coil pipe, and a pulling mechanism portion that applies a compression force to the coil pipe by pulling the wire. The rigidity in the bending direction of the coil pipe is changed depending on the compression force applied thereto. Therefore, the rigidity of the part of the insertion portion in which the coil pipe is inserted is changed depending on the compression force applied to the coil pipe.

In the rigidity changing mechanism portion of the endoscope disclosed in the Japanese Patent Application Laid-Open Publication No. 10-276965, extension of the free length of the wire and contraction of the free length of the coil pipe occur due to repeated changes of the compression force to be applied to the coil pipe. When the extension of the free length of the wire and the contraction of the free length of the coil pipe occur, the relationship between the pulling distance of the wire and the degree of change in the rigidity of the insertion portion changes, which leads to a change in the operation feeling felt by a user.

The present invention solves the above-described point, and an object of the present invention is to provide an endoscope including a rigidity changing mechanism portion in an insertion portion and configured to be capable of preventing extension of a wire and a contraction of a coil pipe.

### Disclosure of Invention

### Means for Solving the Problem

An endoscope according to one aspect of the present invention includes: a flexible tube portion provided to an insertion portion that extends from a hand side to a distal end side along a longitudinal direction; a coil pipe arranged in the flexible tube portion, and formed by being wound around a predetermined axis that is parallel to the longitudinal direction; a first wire inserted in the coil pipe and twined around the predetermined axis in a direction opposite to a winding direction of the coil pipe, a distal end side of the first wire being fixed to a distal end of the coil pipe; a second wire formed by being twined in a direction opposite to a twining direction of the first wire, a proximal end of the second wire being fixed to a portion at which the first wire and the coil pipe are fixed to each other, and a distal end of the second wire being engaged with a constituent element of the endoscope, with rotation of the distal end being restricted; and a pulling mechanism portion that pulls the first wire in a proximal end direction to apply a compression force to the coil pipe.

In addition, an endoscope according to another aspect of the present invention includes: a flexible tube portion provided to an insertion portion that extends from a hand side to a distal end side along a longitudinal direction; a coil pipe arranged in the flexible tube portion, and formed by being wound around a predetermined axis that is parallel to the longitudinal direction; a first wire inserted in the coil pipe and twined around the predetermined axis in a direction opposite to a winding direction of the coil pipe; and a pulling mechanism portion that pulls the first wire in a proximal end direction to apply a compression force to the coil pipe, wherein the coil pipe is arranged in a state being applied with a twisting force in a direction same as the winding direction of the coil pipe.

### Brief Description of the Drawings

FIG. 1 illustrates a configuration of an endoscope according to a first embodiment of the present invention.
FIG. 2 illustrates configurations of a flexible tube portion and a rigidity changing mechanism portion according to the first embodiment.
FIG. 3 illustrates winding directions of a coil pipe, a first wire, and a second wire according to the first embodiment.
FIG. 4 illustrates another example of winding directions of the coil pipe, the first wire, and the second wire according to the first embodiment.
FIG. 5 describes configurations of a flexible tube portion and a rigidity changing mechanism portion according to a second embodiment.

### Best Mode for Carrying Out the Invention

Hereinafter, preferable embodiments of the present invention will be described with reference to drawings. Note that, in the drawings to be used in the description below, a different scale size is used for each of constituent elements in order to allow the each of the constituent elements to be illustrated in a recognizable size, and the present invention is not limited to the number, shapes, ratio of the sizes of the constituent elements, and a relative positional relationship among the constituent elements shown in these drawings.

### (First Embodiment)

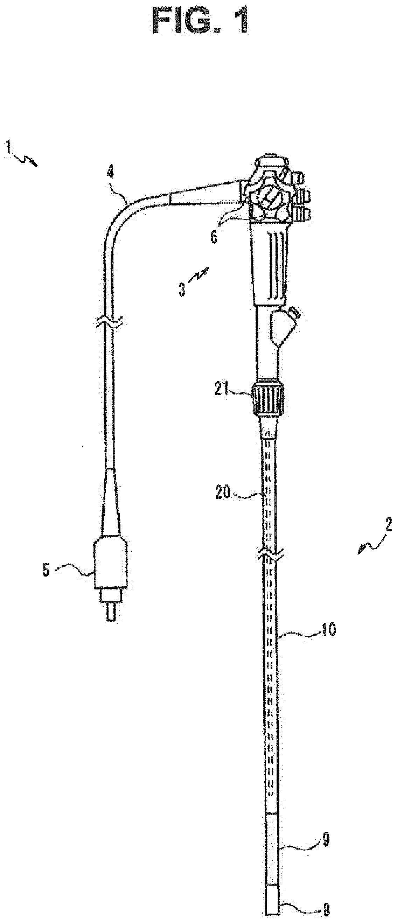

An endoscope 1 according to the present embodiment shown in FIG. 1 includes an elongated insertion portion 2 which is introduceable into a subject such as a human body, and the insertion portion 2 has a configuration for observing the inside of the subject. Note that the subject into which the insertion portion 2 of the endoscope 1 is introduced is not limited to a human body but may be another living body, or an artificial material such as a machine or construction.

The endoscope 1 according to the present embodiment mainly includes the insertion portion 2 formed in an elongated shape and configured to be introduced into an inside of a subject, an operation portion 3 positioned at a proximal end of the insertion portion 2, and a universal cord 4 extended from the operation portion 3.

The insertion portion 2 includes in a linked manner: a distal end portion 8 disposed at a distal end; a bendable bending portion 9 disposed on a proximal end side of the distal end portion 8; and a flexible tube portion 10 having flexibility and connecting the proximal end side of the bending portion 9 and the distal end side of the operation portion 3.

The distal end portion 8 includes a configuration for observing an inside of a subject. For example, the distal end portion 8 includes an image pickup unit including an objective lens and an image pickup device and configured to optically observe the inside of the subject. In addition, the distal end portion 8 is provided with an illumination light emission portion that emits light for illuminating the object of the image pickup unit, though not shown. Note that the distal end portion 8 may be provided with an ultrasound transducer for acoustically observing the inside of the subject using ultrasound.

The operation portion 3 disposed at the proximal end of the insertion portion 2 includes an angle operation knob 6 for operating the bending of the bending portion 9. At the proximal end portion of the universal cord 4, an endoscope connector 5 which is configured to be connectable to an external apparatus, not shown, is provided. The external apparatus to which the endoscope connector 5 is connected includes a camera control unit for controlling the image pickup unit provided at the distal end portion 8, and the like.

In addition, the operation portion 3 is provided with a rigidity changing knob 21 for operating a rigidity changing mechanism portion 20 disposed in the flexible tube portion 10. The rigidity changing mechanism portion 20 is inserted into the flexible tube portion 10 along the longitudinal direction of the flexible tube portion 10, and configured such that the rigidity against flexion changes according to the operation input through the rigidity changing knob 21. That is, the rigidity changing mechanism portion 20 changes the rigidity against the flexion of the flexible tube portion 10.

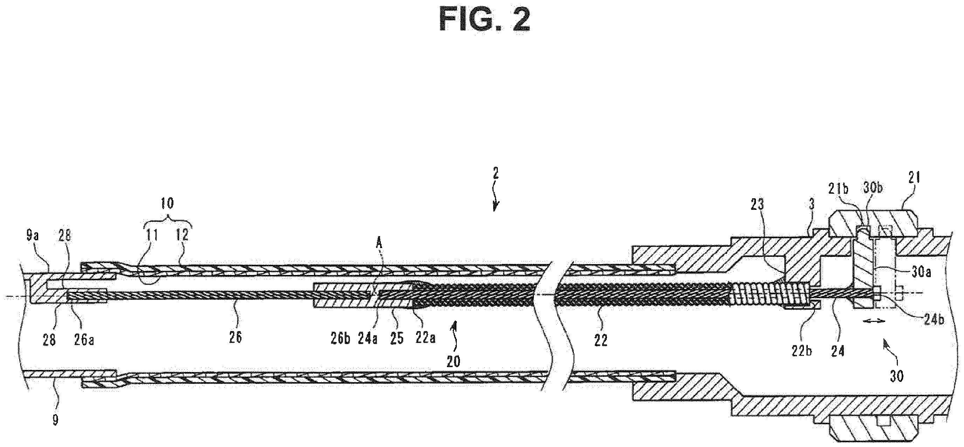

Next, description will be made on the flexible tube portion 10 and the rigidity changing mechanism portion 20. As shown in FIG. 2, the flexible tube portion 10 includes a mesh tube 11 and an outer cover 12.

The mesh tube 11 is formed by braiding a thin wire made of metal such as stainless alloy in a tubular shape. The outer cover 12 is a coat made of synthetic resin that covers the outer circumference of the mesh tube 11. The mesh tube 11 is covered with the outer cover 12, to thereby keep the airtightness in the flexible tube portion 10.

Note that a flex tube which is a core material that prevents crushing of the flexible tube portion 10 is provided inside the mesh tube 11, though not shown. The flex tube is formed by helically winding an elongated thin metal plate around an axis that is along the longitudinal direction of the flexible tube portion 10. The width of the thin plate that configures the flex tube is narrower than a width of a pitch at which the thin plate is wound. The flex tube deforms according to the flexion of the flexible tube portion 10.

Inside the flexible tube portion 10 configured as described above, internal components such as an electric cable that electrically connects the image pickup unit and the endoscope connector and a conduit through which a fluid or a treatment instrument passes are inserted, in addition to the above-described rigidity changing mechanism portion 20. Since the internal components other than the rigidity changing mechanism portion 20 are known techniques, the descriptions thereof will be omitted.

The rigidity changing mechanism portion 20 includes a coil pipe 22, a first wire 24, and a second wire 26. With regard to the members that constitute the rigidity changing mechanism portion 20, the direction toward the distal end portion 8 of the insertion portion 2 is referred to as a distal end direction, and the direction toward the operation portion 3 is referred to as a proximal end direction.

The coil pipe 22 is formed by helically winding a linear member made of metal such as stainless alloy around a predetermined axis A that is parallel to the longitudinal direction of the insertion portion 2.

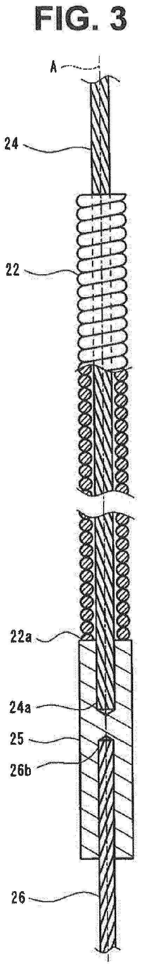

The coil pipe 22 in the present embodiment is formed by winding a linear member into a right-handed helix, as one example as shown in FIG. 3. The right-handed helix is a helix having a winding direction in which when the coil pipe 22 is placed such that the axis A extends in the vertical direction as shown in FIG. 3, the part of the linear member, which is shown on the front side of the coil pipe 22, rises diagonally up to the right. Such a winding direction is also referred to as a Z-winding.

A proximal end 22b of the coil pipe 22 is held by a coil fixing portion 23 fixed to the operation portion 3. The coil pipe 22 includes inside thereof a space having a predetermined internal diameter with the predetermined axis A as a center. The first wire 24 to be described later is inserted in the coil pipe 22.

The first wire 24 is inserted in the coil pipe 22 and formed by twining linear members each other around the predetermined axis A in a direction opposite to the winding direction of the coil pipe 22. The first wire 24 is formed by twining the linear members made of metal such as stainless alloy each other, for example.

In the present embodiment, the coil pipe 22 is the right-handed helix as described above. Therefore, the first wire 24 is formed by twining the linear members into a left-handed helix. The left-handed helix is a helix having a winding direction in which when the first wire 24 is placed such that the axis A extends in the vertical direction as shown in FIG. 3, the part of the linear members, which is shown on the front side of the first wire 24, rises diagonally up to the left. Such a winding direction is also referred to as S-winding, S-twining, or the like.

A distal end 24a of the first wire 24 inserted in the coil pipe 22 is engaged with a distal end 22a of the coil pipe 22 such that a compression force in a direction in which the coil pipe 22 is compressed along the axis A is applied to the coil pipe 22, when the first wire 24 is pulled in the proximal end direction.

Specifically, the distal end 24a of the first wire 24 protrudes further in the distal end direction than the distal end 22a of the coil pipe 22. A connection portion 25 having an outer shape larger than the inner diameter of the coil pipe 22 is secured to the distal end 24a of the first wire 24. That is, the relative movement of the distal end 24a of the first wire 24 in the proximal end direction with respect to the distal end 22a of the coil pipe 22 is restricted by the connection portion 25.

In addition, the connection portion 25 is secured to the distal end 22a of the coil pipe 22 by adhesive, soldering, brazing, or the like. That is, the distal end 24a of the first wire 24 is fixed to the distal end 22a of the coil pipe 22. Note that the distal end 24a of the first wire 24 may be directly secured to the distal end 22a of the coil pipe 22 by adhesive, soldering, brazing, or the like without using the connection portion 25.

As described above, the proximal end 22b of the coil pipe 22 is fixed to the operation portion 3 by the coil fixing portion 23. Therefore, when the first wire 24 is pulled in the proximal end direction, a tensile force applied to the first wire 24 is transferred to the distal end 22a of the coil pipe 22, and the force for compressing the coil pipe 22 in the direction of the axis A is applied to the coil pipe 22. The compression force is applied to the coil pipe 22, to thereby increase a resistance force against the bending deformation, which is generated by the coil pipe 22. The greater the compression force to be applied to the coil pipe 22, the greater the resistance force against the bending deformation generated by the coil pipe 22.

The proximal end 24b of the first wire 24 is connected to a pulling mechanism portion 30 that pulls the first wire 24 in the proximal end direction and applies the tensile force to the first wire 24.

Since the pulling mechanism portion 30 is known, detailed description thereof will be omitted. In the present embodiment, as one example, the pulling mechanism portion 30 includes a rigidity changing knob 21 that rotates with respect to the operation portion 3, and a wire holding portion 30a that holds the proximal end 24b of the first wire 24 and advances and retracts in the direction along the axis A according to the rotation of the rigidity changing knob 21.

A cam groove 21b is carved on the inner circumferential surface of the rigidity changing knob 21. The wire holding portion 30a is disposed so as to be able to advance and retract in the direction along the axis A in the operation portion 3. In addition, the wire holding portion 30a is provided with a cam pin 30b that is slidably engaged with the cam groove 21b. Engagement between the cam groove 21b and the cam pin 30b allows the wire holding portion 30a to advance and retract in the direction along the axis A according to the rotation of the rigidity changing knob 21. The pulling mechanism portion 30 thus configured according to the present embodiment is capable of changing the tensile force to be applied to the first wire 24 in accordance with the rotation operation of the rigidity changing knob 21 by the user.

The second wire 26 is formed by winding linear members around the predetermined axis A in the direction opposite to the twining direction of the first wire 24. That is, the second wire 26 is formed by twining the linear members around the predetermined axis A in the direction same as the winding direction of the coil pipe 22. The second wire 26 is formed by twining the linear members made of metal such as stainless alloy each other, for example.

In the present embodiment, as described above, the first wire 24 is twined in a left-handed fashion. Therefore, the second wire 26 is formed by twining the linear members into the right-handed helix. The right-handed twining is also referred to as Z-winding or Z-twining.

The second wire 26 is fixed to the constituent element of the insertion portion 2, with the rotation of the distal end 26a being restricted, and the proximal end 26b is fixed to the distal end 24a of the first wire 24. That is, the second wire 26 is arranged on the distal end side with respect to the first wire 24.

Specifically, the distal end 26a of the second wire 26 is fixed to a wire fixing portion 28 of a frame member 9a arranged at the proximal end of the bending portion 9 of the insertion portion 2, with the rotation around the axis A being restricted. The distal end 26a of the second wire 26 is secured to the wire fixing portion 28 by adhesive, soldering, brazing, or the like, for example.

In addition, the proximal end 26b of the second wire 26 is secured to the connection portion 25 by adhesive, soldering, brazing, or the like, for example. The distal end 24a of the first wire 24 and the proximal end 26b of the second wire 26 are secured to the connection portion 25 such that the central axes thereof are located on the axis A.

Note that the proximal end 26b of the second wire 26 may be directly secured to the distal end 24a of the first wire 24 by adhesive, soldering, brazing, or the like, for example, without using the connection portion 25.

The distal end 26a of the second wire 26 is secured to the frame member 9a of the bending portion 9, which allows the position of the distal end 24a of the first wire 24 in the flexible tube portion 10 to be held within a predetermined range. That is, the range in which the distal end 22a of the coil pipe 22 can be moved in the flexible tube portion 10 is determined by the second wire 26. Therefore, the coil pipe 22 is held so as to be movable in the flexible tube portion 10 only in the range determined by the second wire 26 in the case where the first wire 24 is pulled in the proximal end direction by the pulling mechanism portion 30 and flexion of the flexible tube portion 10 is repeated.

The resistance force of the coil pipe 22 against the bending deformation changes according to the tensile force applied to the first wire 24 by the pulling mechanism portion 30. Therefore, the rigidity against the flexion of the flexible tube portion 10 in the range in which the coil pipe 22 is arranged changes according to the resistance force against the bending deformation of the coil pipe 22. According to the configuration as described above, the rigidity changing mechanism portion 20 changes the rigidity of at least a part of the insertion portion 2.

As described above, the rigidity changing mechanism portion 20 included in the endoscope 1 according to the present embodiment is provided with the coil pipe 22 formed by winding the linear member around the predetermined axis A, the first wire 24 twined around the axis A in the direction opposite to the winding direction of the coil pipe 22, the second wire 26 twined in the direction opposite to the twining direction of the first wire 24, and the pulling mechanism portion 30 that pulls the first wire 24.

In addition, the first wire 24 is inserted in the coil pipe 22, the distal end 24a of the first wire 24 is fixed to the distal end 22a of the coil pipe 22, and the proximal end 24b of the first wire 24 is connected to the pulling mechanism portion 30. In addition, the second wire 26 is engaged with the constituent element of the insertion portion 2, with the rotation of the distal end 26a being restricted, and the proximal end 26b is fixed to a portion at which the first wire 24 and the coil pipe 22 are fixed to each other. The pulling mechanism portion 30 applies a compression force to the coil pipe 22 by pulling the first wire 24 in the proximal end direction.

In the rigidity changing mechanism portion 20 thus configured according to the present embodiment, when the tensile force is applied to the first wire 24 by the pulling mechanism portion 30, the first wire 24 generates a rotational force F1 around the axis A by being untwined. The deformation due to the untwining of the first wire 24 can be reworded as the deformation due to extension of the first wire 24.

On the other hand, when the tensile force is applied to the first wire 24 by the pulling mechanism portion 30, the compression force is applied to the coil pipe 22, and the coil pipe 22 generates a rotational force F2 around the axis A by being deformed so that the total number of windings of the coil pipe 22 is decreased. The deformation due to the decrease in the total number of windings of the coil pipe 22can be reworded as the deformation due to the contraction of the coil pipe 22.

The winding direction of the coil pipe 22 and the twining direction of the first wire 24 are in the relationship opposite to each other. When the tensile force is applied to the first wire 24 by the pulling mechanism portion 30, the rotational force F1 generated by the first wire 24 and the rotational force F2 generated by the coil pipe 22 are in a relationship in which the rotational forces act in directions so as to cancel each other. Therefore, the rigidity changing mechanism portion 20 according to the present embodiment is capable of suppressing or preventing the amount of deformation that occurs when the coil pipe 22 contracts and the amount of deformation that occurs when the first wire 24 extends, when a tensile force is applied to the first wire 24, thereby being capable of suppressing or preventing the contraction of the free length of the coil pipe 22 and the extension of the free length of the first wire 24 which are caused by repeated use.

In addition, the rigidity changing mechanism portion 20 according to the present embodiment includes the second wire 26 twined in the direction opposite to the twining direction of the first wire 24. When the first wire 24 is pulled by the pulling mechanism portion 30 in the proximal end direction, a tensile force is applied also to the second wire 26. Since the second wire 26 is in the state where the rotation of the distal end 26a is restricted, when the proximal end 26b is pulled in the proximal end direction, the second wire 26 generates a rotational force F3 around the axis A by being untwined.

The twining direction of the first wire 24 and the twining direction of the second wire 26 are in the relationship opposite to each other. Therefore, when the tensile force is applied to the first wire 24 by the pulling mechanism portion 30, the rotational force F1 generated by the first wire 24 and the rotational force F3 generated by the second wire 26 are in the relationship in which the rotational forces act in directions so as to cancel each other. The rigidity changing mechanism portion 20 according to the present embodiment is capable of suppressing or preventing the change in the twining of the first wire 24 and second wire 26 when the tensile force is applied to the first wire 24. As a result, extension of the free lengths of the first wire 24 and the second wire 26, which is caused by the repeated use, can be suppressed or prevented.

In addition, the rigidity changing mechanism portion 20 according to the present embodiment is capable of suppressing or preventing the change in the twining of the first wire 24 and second wire 26 when the tensile force is applied to the first wire 24, thereby capable of preventing kink caused by the second wire 26 being untwined too much or twined too tightly.

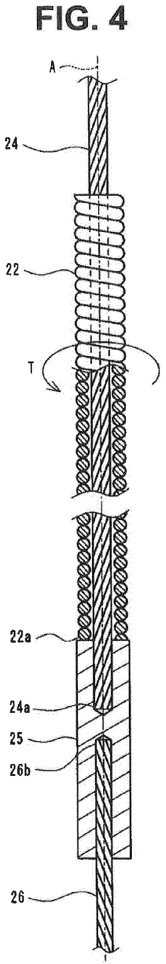

Note that, in the present embodiment described above, the coil pipe 22 and the second wire 26 are right-handed (Z-winding), and the first wire 24 is left-handed (S-winding). However, the winding directions may be reversed. That is, as shown in FIG. 4, even if the coil pipe 22 and the second wire 26 are left-handed (S-winding) and the first wire 24 is right-handed (Z- winding), the same working and effects as those in the above-described embodiment can be obtained.

### (Second Embodiment)

Hereinafter, the second embodiment of the present invention will be described. Hereinafter, only the points different from the first embodiment will be described. The same constituent elements as those in the first embodiment are attached with the same reference numerals and descriptions thereof will be omitted.

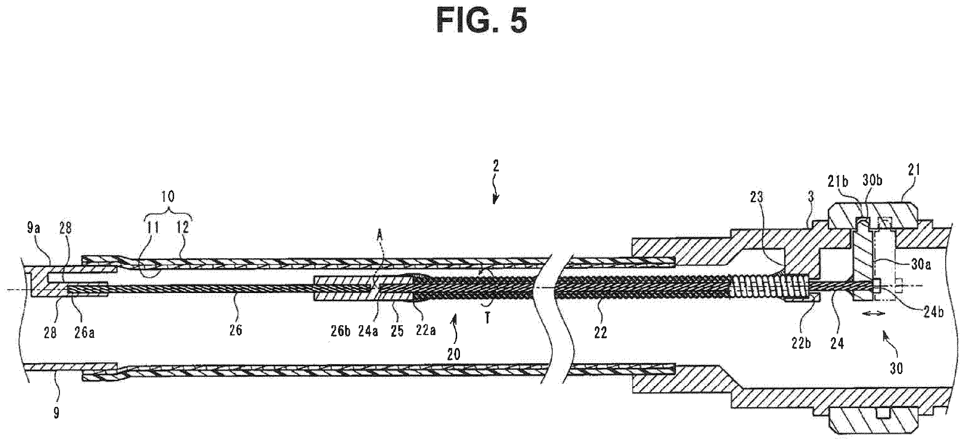

The endoscope 1 according to the present embodiment shown in FIGS. 4 and 5 is different from the one in the first embodiment in that the coil pipe 22 of the rigidity changing mechanism portion 20 is applied with a twisting force T around the axis A in a direction same as the winding direction of the coil pipe 22.

In the present embodiment shown in the drawings, as one example, the coil pipe 22 is formed by winding a linear member right-handedly (Z winding). The coil pipe 22 according to the present embodiment is configured such that the distal end 22a is fixed to the distal end 24a of the first wire 24 and the proximal end 22b is fixed to the coil fixing portion 23, in the state where the coil pipe 22 is twisted by a predetermined angle from the natural state in the direction same as the winding direction.

Twisting the coil pipe 22 in the direction same as the winding direction means that twisting the coil pipe 22 around the axis A in the direction in which the total number of windings of the coil pipe 22 increases. Arranging the coil pipe 22 in the state twisted in the direction same as the winding direction thereof causes the resistance force against the bending deformation, which is generated by the coil pipe 22, to be greater than in the case where the coil pipe 22 is in the natural state.

Therefore, in the present embodiment, the initial rigidity of the coil pipe 22 in the state where the first wire 24 is not pulled by the pulling mechanism portion 30 can be adjusted by changing the magnitude of the twisting force T to be applied to the coil pipe 22. Adjusting the initial rigidity of the coil pipe 22 enables the variation of the rigidities of the flexible tube portions 10 which occurs in a plurality of endoscopes 1 to be suppressed.

In addition, in the present embodiment, similarly as in the first embodiment, the winding direction of the coil pipe 22 is same as the winding direction of the second wire 26, and the winding direction of the first wire 24 is opposite to the winding direction of the coil pipe 22. Therefore, also in the endoscope 1 according to the present embodiment, similarly as in the first embodiment, when the tensile force is applied to the first wire 24, the amount of deformation that occurs when the coil pipe 22 contracts and the amount of deformation that occurs when the first wire 24 extends can be suppressed or prevented, and the contraction of the free length of the coil pipe 22 and the extension of the free length of the first wire 24 can be suppressed or prevented. In addition, the endoscope according to the present embodiment is capable of suppressing or preventing the change in the twining of the first wire 24 and second wire 26 when the tensile force is applied to the first wire 24, thereby capable of preventing kink caused by the second wire 26 being untwined too much or twined too tightly.

The present invention is not limited to the above-described embodiments, and appropriate changes are possible without departing from the gist or thought of the invention that can be read from claims and throughout the specification, and endoscopes with such changes are also included in the technical range of the present invention.

The present application is filed claiming priority from Japanese Patent Application No. 2015-224987 filed in Japan on November 17, 2015, disclosed contents of which are incorporated in this specification, claims, and drawings by reference.

## Claims

1. An endoscope comprising:
a flexible tube portion provided to an insertion portion that extends from a hand side to a distal end side along a longitudinal direction;
a coil pipe arranged in the flexible tube portion, and formed by being wound around a predetermined axis that is parallel to the longitudinal direction;
a first wire inserted in the coil pipe and twined around the predetermined axis in a direction opposite to a winding direction of the coil pipe, a distal end side of the first wire being fixed to a distal end of the coil pipe;
a second wire formed by being twined in a direction opposite to a twining direction of the first wire, a proximal end of the second wire being fixed to a portion at which the first wire and the coil pipe are fixed to each other, and a distal end of the second wire being engaged with a constituent element of the endoscope, with rotation of the distal end being restricted; and
a pulling mechanism portion that pulls the first wire in a proximal end direction to apply a compression force to the coil pipe.

2. An endoscope comprising:
a flexible tube portion provided to an insertion portion that extends from a hand side to a distal end side along a longitudinal direction;
a coil pipe arranged in the flexible tube portion, and formed by being wound around a predetermined axis that is parallel to the longitudinal direction;
a first wire inserted in the coil pipe and twined around the predetermined axis in a direction opposite to a winding direction of the coil pipe; and
a pulling mechanism portion that pulls the first wire in a proximal end direction to apply a compression force to the coil pipe,
wherein the coil pipe is arranged in a state being applied with a twisting force in a direction same as the winding direction of the coil pipe.

3. The endoscope according to claim 2, further comprising a second wire formed by being twined in a direction opposite to a twining direction of the first wire, a proximal end of the second wire being fixed to a portion at which the first wire and the coil pipe are fixed to each other, and a distal end of the second wire being engaged with a constituent element of the endoscope, with rotation of the distal end being restricted.
